# FASCICULE DE BREVET EUROPEEN

(11) **EP 1 006 859 B1**
(45) Date de publication et mention de la délivrance du brevet: **03.12.2003**
(21) Numéro de dépôt: 97942068.4
(22) Date de dépôt: 19.09.1997
(51) Int. Cl.: A61B 1/32

(54) **SPECULUM, NOTAMMENT UN SPECULUM VAGINAL A USAGE UNIQUE**
SPEKULUM,INSBESONDERE VAGINAL-SPEKULUM ZUR EINMALIGEN VERWENDUNG
SPECULUM, IN PARTICULAR VAGINAL SPECULUM FOR SINGLE USE

(30) Priorité: 24.09.1996 FR 9611621
(43) Date de publication de la demande: 14.06.2000
(73) Titulaire: Central Labo Europe (S.a.r.l.), 75002 Paris (FR)
(72) Inventeur: BARA, Nicolas, 75002 Paris (FR); JIPPE, Claude, 75002 Paris (FR); DIETRICH, Emmanuel, 75002 Paris (FR); LAUZET, Maurice, 75002 Paris (FR)
(74) Mandataire: Breese, Pierre
(86) Numéro de dépôt international: FR9701661
(87) Numéro de publication internationale: WO98012961

(56) Documents cités:
- WO-A-92/00698
- DE-U- 9 209 244
- US-A- 3 847 143
- US-A- 4 966 130

## Description

La présente invention concerne un spéculum, notamment un spéculum vaginal à usage unique. Les spéculums sont destinés à l'examen du col de l'utérus féminin, par examen visuel ou à l'aide d'instruments optiques.

On connaissait dans l'état de la technique des spéculums métalliques qui doivent être stérilisés après chaque usage. Leur coût de fabrication est élevé, et leur utilisation implique une main d'oeuvre pour le nettoyage et la stérilisation.

On a également proposé de réaliser des spéculums à usage unique en matière plastique moulée. De tels spéculums sont généralement réalisés sous forme de deux organes d'écartements raccordés par des pivots, et complétés par des organes de blocage constitués par une vis ou une crémaillère. Ce moyen de blocage est destiné à maintenir un degré d'ouverture correspondant à l'une des positions de blocage possible, les organes d'écartement étant soumis à la tension des tissus. Un tel dispositif est décrit dans le brevet français FR7925424. Le coût de fabrication de tels spéculums à usage unique est élevé car la fabrication implique des investissements élevés pour la réalisation d'une pluralité de moules, et nécessite plusieurs opérations d'assemblages.

On a également proposé dans l'état de la technique des spéculums monopièces.

Un premier exemple de spéculum monopièce a été décrit dans le brevet PCT WO92/00698. Ce spéculum monopièce est formé de deux organes d'écartement moulés "tête-bêche", reliés par des pontets de matière plastique. Les deux organes d'écartement sont prolongés à la partie arrière de moyens complémentaires raccordables pour former une charnière. La réalisation du spéculum implique des opérations d'assemblage consistant à basculer les organes d'écartement l'un sur l'autre, la pénétration relative des moyens complémentaires pour former la charnière et l'ébavurage du pontet. Ce type de spéculum ne permet pas de supprimer totalement les opérations d'assemblage, et nécessite un usinage soigneux pour éviter de laisser subsister des arêtes susceptibles de provoquer des blessures des tissus vaginaux.

Un autre document de l'art antérieur a été divulgué par le brevet US4263898.

Ce brevet décrit un spéculum monopièce présentant une section constante le long d'un axe passant par l'axe de pivotement des deux branches d'écartement. Une telle réalisation interdit bien sûr la réalisation de branches semi-cylindriques épousant la cavité vaginale de manière peu invasive. Ce dispositif n'est donc pas satisfaisant.

On connaît également des brevets US4966130 et US 3847143 un spéculum moulé en une seule pièce en matière plastique et qui comporte deux branches d'écartement mobiles par pivotement l'un par rapport à l'autre, l'une des branches d'écartement étant prolongé par un élément de préhension. Le spéculum comporte en outre des moyens permettant de contrôler le pivotement des branches d'écartement.

Le spéculum objet de ces deux brevets présente cependant l'inconvénient d'offrir une mise en oeuvre relativement peu aisée et restant relativement invasive.

L'objet de la présente invention est de proposer un spéculum monopièce permettant une fabrication par moulage sans qu'il ne soit nécessaire de prévoir des opérations d'usinage ou d'assemblage supplémentaire, et qui présente une géométrie garantissant un emploi aussi peu invasif que possible.

A cet effet, l'invention concerne plus généralement un spéculum monopièce moulé en matière plastique comprenant deux branches d'écartement en forme de becs de canard, mobiles par pivotement l'une par rapport à l'autre, chacune de ces branches d'écartement étant prolongée par un élément de préhension, le spéculum comportant en outre un moyen de blocage du pivotement des branches d'écartement, caractérisé en ce que les deux branches d'écartement sont reliées par deux segments latéraux s'étendant dans le plan transversal passant par l'extrémité arrière du plan de joint desdites branches d'écartement, et en ce que l'une des branches d'écartement est munie d'un élément de préhension formé par une partie rabattable mobile entre une position de moulage sensiblement perpendiculaire à l'axe longitudinal de ladite branche, et une position d'utilisation sensiblement dans le plan longitudinal de ladite branche.

De préférence, la partie rabattable présente une découpe de forme complémentaire à l'extrémité arrière de la branche d'écartement.

Selon un mode de réalisation avantageux, la partie rabattable s'appuie, en position d'utilisation, sur des ailettes latérales prévues sur la surface d'extérieure de la branche d'écartement.

Selon une autre variante avantageuse, l'une des branches est prolongée par un manchon s'étendant sensiblement perpendiculairement à l'axe de la branche.

Selon un mode de mise en oeuvre particulier, les moyens de blocage sont formés par une languette crantée s'étendant depuis l'extrémité de l'une des branches en direction de l'extrémité de l'autre branche munie d'une surface d'appui propre à coopérer avec lesdits crantages.

Avantageusement, la partie rabattable présente un coin saillant propre à verrouiller les moyens de blocage en position de fermeture lorsqu'elle est rabattue en position d'utilisation.

Avantageusement, la surface extérieure des coins vient s'encastrer dans la cavité, en venant en contact avec la surface intérieure de l'extrémité des languettes crantées et en ce que, au moment du retrait, la surface intérieure des coins vient s'appuyer sur la surface extérieure de l'extrémité des languettes crantées, afin de faciliter le déverrouillage en évitant les à-coups lors du retrait de l'instrument.

Avantageusement, le coin prévu sur la partie rabattable présente une rainure dont le flanc extérieur est en biais afin de pouvoir prendre appui sur le bord extérieur de l'extrémité de la languette crantée, et repousser vers l'intérieur cette extrémité de façon à provoquer le déverrouillage des branches.

De préférence, les branches d'écartement présentent, à l'extrémité avant, une protubérance faisant saillie par rapport à la surface intérieure pour éviter une fermeture complète desdites branches.

Avantageusement, le plan de joint des branches d'écartement respectivement est légèrement bombé et présente une ligne de joint de moulage située vers l'intérieur des branches.

Selon un mode de réalisation préféré, le spéculum est constitué en une matière plastique autolubrifiée, par exemple par une charge d'huile ou d'une matière analogue migrant vers la surface de la pièce ou par un revêtement tel que du TEFLON (nom commercial) déposé sur les surfaces extérieures par projection ou par passage dans un bain ou encore par traitement par plasma froid.

L'invention sera mieux comprise à la lecture de la description qui suit, faisant référence à un mode non limitatif de réalisation, illustré par les dessins annexés où :
- la figure 1 représente une vue éclatée d'un spéculum selon l'invention ;
- la figure 2 représente une vue de la partie arrière du spéculum, en position de moulage :
- la figure 3 représente une vue de la partie arrière du spéculum, en position d'utilisation ;
- la figure 4 représente une vue en section d'une variante de réalisation ;
- les figures 5 à 8 représentent une vue en détail du système de blocage, à différents stades d'utilisation.

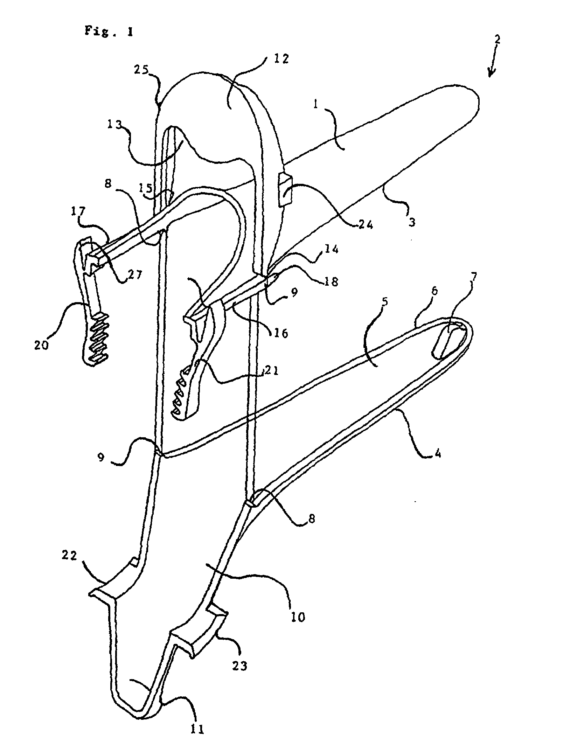

La figure 1 représente un exemple de réalisation d'un spéculum selon l'invention, en vue éclatée.

Il est constitué par une seule pièce moulée en matière plastique.

Il présente une branche d'écartement supérieure (1) en forme de bec de canard. Cette branche d'écartement (1) présente une surface extérieure correspondant sensiblement à un demi-cône, convergeant vers la partie avant (2), et une surface intérieure également en forme de tuile semi-conique. L'épaisseur de la paroi est d'environ 2 millimètres. Les parois se terminent par un plan de joint (3) .

La branche d'écartement inférieure (4) présente une forme sensiblement symétrique à la branche supérieure (1). Elle présente également une forme générale en bec de canard, avec une surface extérieure semi-conique et une surface intérieure (5) en forme de tuile semi-conique, se terminant par un plan de joint (6).

La surface intérieure de chacune des branches d'écartement (1, 4) présente, à l'avant, une protubérance (7,7') empêchant la fermeture complète des deux branches. La hauteur des protubérances (7, 7') est déterminée de façon à ce que l'angle de fermeture maximal des branches soit atteint lorsque les deux protubérances viennent en contact et lorsque les deux plans de joint (3, 6) sont sensiblement parallèles. Ceci évite le pincement des tissus au moment du déblocage du spéculum et de son retrait.

Les deux branches d'écartement (1, 4) présentent à leurs parties arrières des prolongements (8, 9) formant des segments souples s'étendant sensiblement dans le plan transversal. Ces segments sont formés par des zones moulées minces déterminées de façon à autoriser un basculement relatif des branches d'écartement (1, 4) sans cassure, du moins avant un nombre de manipulations raisonnables pour un instrument à usage unique. Ces segments (8, 9) sont réalisés par moulage, à l'aide d'un moule présentant une partie fixe correspondant au profil extérieure des deux branches d'écartement (1, 4) et comportant une langue médiane correspondant à l'espace subsistant entre les deux plans de joint (3, 6) et s'étendant jusqu'à la surface avant des segments (8, 9). Le moule comporte par ailleurs un tiroir mobile s'étendant jusqu'à la surface arrière desdits segments (8, 9). Le plan de coupe du moule est formé par une succession de plans sensiblement transversaux et correspond sensiblement à la séparation entre la partie avant du spéculum, et la partie arrière.

Selon un mode de fabrication préféré, la ligne de raccordement des deux plaques se situe, pour deux branches d'écartement, en arrière de la surface extérieure, afin d'éviter la formation d'une arête blessante. Le plan de joint (3, 6) des branches d'écartement respectivement (1, 4) est légèrement arrondie et bombée avec une ligne de joint de moulage située vers l'intérieur des branches (1, 4).

La branche inférieure (4) est prolongée par un manche (10) formant un angle ad-hoc avec l'axe de la branche, soit environ 60°. Ce manche (10) présente une griffe (11) permettant le raccordement d'une source lumineuse. Cette source lumineuse peut être constituée par un boîtier comportant une source électrique et une ampoule électrique projetant un faisceau lumineux dans l'axe médian des branches (1, 4), ou encore par une fibre optique.

La branche d'écartement supérieure (1) présente à sa partie arrière une partie rabattable (12) s'étendant sensiblement perpendiculairement au plan de joint (3), au moment du moulage. Ladite partie rabattable (12) présente une découpe (13) de forme complémentaire à la partie arrière de la branche (1). Elle est reliée à la surface extérieure de la branche (1) par des zones d'affaiblissement latérales (14, 15) formant une charnière. Des ailettes latérales (16, 17) forment un appui de la partie rabattable (12). Ces ailettes se prolongent à l'avant de la charnière (14, 15) par un bec (18) empêchant le basculement vers l'avant au delà de la position de moulage.

La partie arrière de la branche supérieure (1) comporte des languettes (20, 21) crantées s'étendant dans le plan transversal.

Ces languettes (20, 21) viennent coopérer avec des surfaces d'appui (22, 23) courbes prolongeant latéralement le manche (10) de la branche inférieure (4).

La partie rabattable (12) présente des coins latéraux (24, 25) venant se loger dans des cavités complémentaires (26, 27) pour renforcer le blocage, lorsque la partie rabattable (12) est rabattue en position d'utilisation.

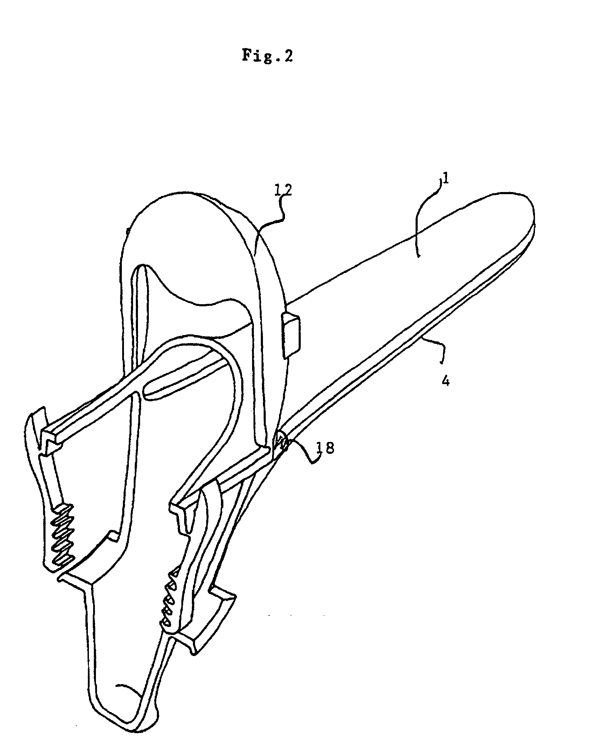

La figure 2 représente une vue en perspective, de 3/4 arrière, du spéculum avant utilisation. La partie rabattable (12) est en position verticale. Elle est bloquée vers l'avant par le bec (18).

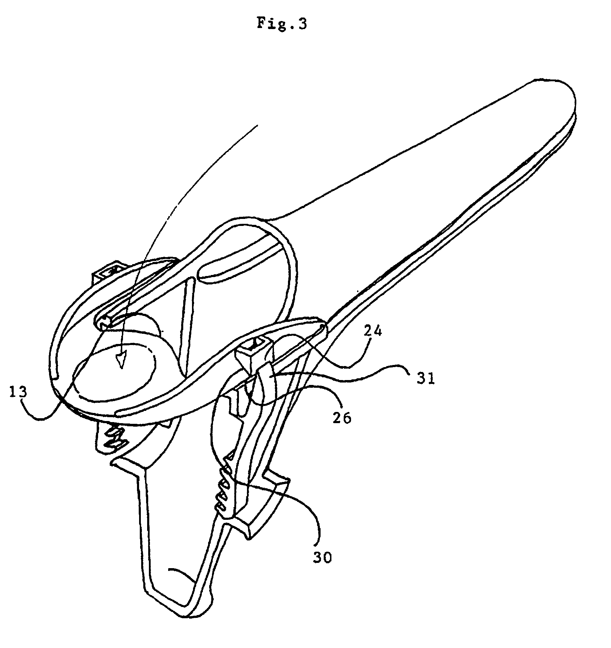

Pour utiliser le spéculum, on bascule la partie rabattable (12) comme représenté sur la figure 3. Le bord de la découpe (13) vient s'appuyer sur les ailettes latérales (16, 17) de la branche supérieure (1), ce qui permet au praticien d'exercer avec son pouce une pression suffisante pour écarter les tissus s'appuyant sur les branches (1, 4). La surface extérieure des coins (24, 25) vient s'encastrer dans la cavité (26, 27), en venant en contact avec la surface intérieure de l'extrémité des languettes crantées (20, 21) .

Au moment du retrait, c'est au contraire la surface intérieure (30) des coins (24, 25) qui vient s'appuyer sur la surface extérieure (31) de l'extrémité des languettes crantées (20, 21), afin de faciliter le déverrouillage en évitant les à-coups lors du retrait de l'instrument.

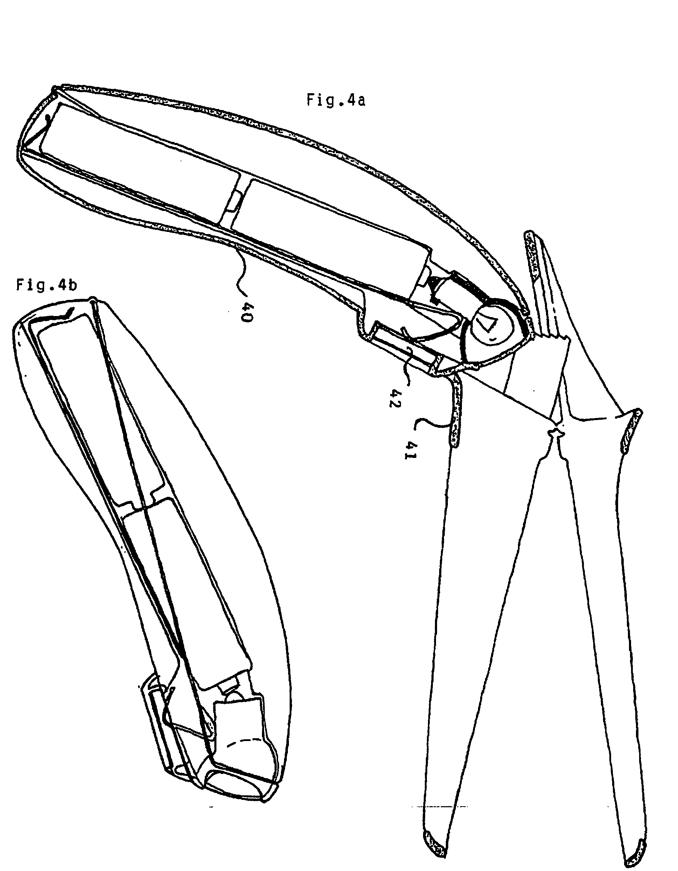

La figure 4 représente une vue en section d'une variante de réalisation. Dans cette variante, le spéculum est prolongé par un boîtier de lampe (40) se prolongeant par une partie (41) venant s'appuyer sur la surface inférieure arrière de la branche inférieure (4). Le raccordement entre le spéculum à usage unique et le boîtier de lampe s'effectue à l'aide d'une griffe (42). Ce boîtier présente une forme ergonomique pour faciliter la prise en main du spéculum.

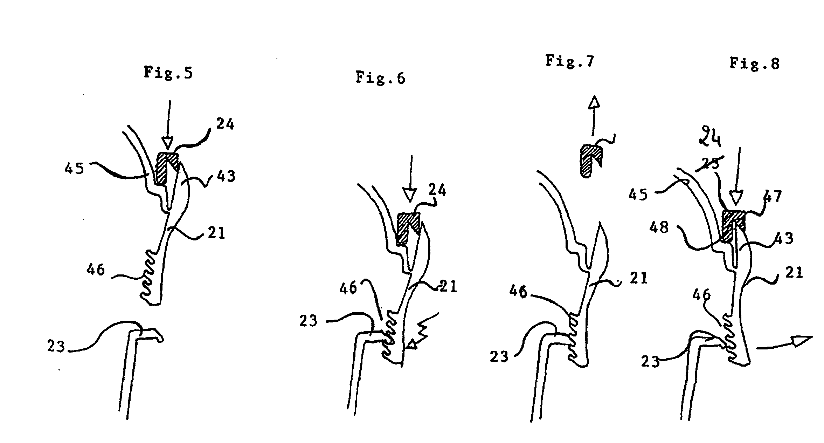

Les figures 5 à 8 représentent des vues en détail du système de blocage à différents stades d'utilisation. Lorsque la partie rabattable (12) est abaissée, le coin (24) vient se loger dans l'espace compris entre l'extrémité supérieure (43) de la languette crantée (21), et le corps (45) du spéculum. De ce fait, la zone crantée (46) de la languette crantée (21) est repoussée vers l'intérieur. Lorsque l'utilisateur appuie sur la partie rabattable (12) pour écarter les branches du spéculum, la zone crantée (46) vient en contact avec la surface d'appui (23). Le blocage est renforcé par la contrainte exercée par le coin (24).

Pendant l'examen gynécologique, la partie rabattable (12) est relevée pour éviter de masquer la visibilité. Le verrouillage des branches en position écartée est maintenue par l'action de la zone crantée (46) sur la surface d'appui (23).

Après l'examen, le praticien abaisse à nouveau la partie rabattable (12). La zone crantée (46) étant toujours en appui sur la surface d'appui (23), l'extrémité supérieure (43) de la languette crantée (21) est légèrement écartée du corps (45) du spéculum. Le coin (24) présente une rainure (48) présentant un flanc extérieur (47) en biais. Ce flanc vient prendre appui sur le bord extérieur de l'extrémité (43) de la languette crantée (21), et vient repousser vers l'intérieur cette extrémité (43). La zone crantée (46) est alors légèrement écartée de la surface d'appui (23), ce qui provoque le déverrouillage des branches (1, 4) qui peuvent se rapprocher et permettre le retrait du spéculum.

L'invention est décrite dans ce qui précède à titre d'exemple non limitatif. Il est bien évident que l'Homme de Métier sera à même de réaliser des variantes sans pour autant sortir du cadre de l'invention.

## Revendications

1. Spéculum monopièce moulé en matière plastique comprenant deux branches d'écartement (1, 4) en forme de becs de canard, mobiles par pivotement l'une par rapport à l'autre, chacune desdites branches d'écartement étant prolongée par un élément de préhension, le spéculum comportant en outre un moyen de blocage du pivotement des branches d'écartement,
**caractérisé en ce que** les deux branches d'écartement (1, 4) ne sont reliées que par deux segments latéraux (8, 9) s'étendant dans le plan transversal passant par l'extrémité arrière du plan de joint (3,6) desdites branches d'écartement (1, 4)
et **en ce que** l'une des branches d'écartement (1, 4) est munie d'un élément de préhension formé par une partie rabattable (12) mobile entre une position de moulage sensiblement perpendiculaire à l'axe longitudinal de ladite branche, et une position d'utilisation sensiblement dans le plan longitudinal de ladite branche.

2. Spéculum monopièce selon la revendication 1 **caractérisé en ce que** la partie rabattable (12) présente une découpe de forme complémentaire à l'extrémité arrière de la branche d'écartement.

3. Spéculum monopièce selon la revendication 1 ou 2 **caractérisé en ce que** la partie rabattable (12) s'appuie, en position d'utilisation, sur des ailettes latérales prévues sur la surface d'extérieure de la branche d'écartement.

4. Spéculum monopièce selon l'une quelconque des revendications précédentes **caractérisé en ce que** l'une des branches (1, 4) est prolongée par un manchon s'étendant sensiblement perpendiculairement à l'axe de la branche.

5. Spéculum monopièce selon l'une quelconque des revendications précédentes **caractérisé en ce que** les moyens de blocage sont formés par une languette crantée (20, 21) s'étendant depuis l'extrémité de l'une des branches en direction de l'extrémité de l'autre branche munie d'une surface d'appui (22, 23) propre à coopérer avec lesdits crantages.

6. Spéculum monopièce selon la revendication 5 **caractérisé en ce que** la partie rabattable (12) présente un coin (24, 25) saillant propre à verrouiller les moyens de blocage en position de fermeture lorsqu'elle est rabattue en position d'utilisation.

7. Spéculum monopièce selon la revendication 6 **caractérisé en ce que** la surface extérieure des coins (24, 25) vient s'encastrer dans la cavité (26, 27), en venant en contact avec la surface intérieure de l'extrémité des languettes crantées (20, 21) et **en ce que**, au moment du retrait, la surface intérieure (30) des coins (24, 25) vient s'appuyer sur la surface extérieure (31) de l'extrémité des languettes crantées (20, 21), afin de faciliter le déverrouillage en évitant les à-coups lors du retrait de l'instrument.

8. Spéculum monopièce selon la revendication 6 ou 7 **caractérisé en ce que** le coin (24) prévu sur la partie rabattable (12) présente une rainure (48) dont le flanc extérieur (47) est en biais afin de pouvoir prendre appui sur le bord extérieur de l'extrémité (43) de la languette crantée (21), et repousser vers l'intérieur cette extrémité (43) de façon à provoquer le déverouillage des branches (1, 4).

9. Spéculum monopièce selon l'une quelconque des revendications précédentes **caractérisé en ce que** les branches d'écartement (1, 4) présentent, à l'extrémité avant, une protubérance (7, 7') faisant saillie par rapport à la surface intérieure pour éviter une fermeture complète desdites branches.

10. Spéculum monopièce selon l'une quelconque des revendications précédentes **caractérisé en ce que** le plan de joint (3, 6) des branches d'écartement respectivement (1, 4) est légèrement bombé et présente une ligne de joint de moulage située vers l'intérieur des branches (1, 4).

11. Spéculum monopièce selon l'une quelconque des revendications précédentes **caractérisé en ce qu'**il est constitué en une matière plastique autolubrifiée.

12. Spéculum monopièce selon la revendication 11 **caractérisé en ce que** la matière plastique est lubrifiée par incorporation dans la masse d'un additif migrant vers la surface de la pièce.

13. Spéculum monopièce selon la revendication 11 **caractérisé en ce que** la matière plastique est lubrifiée par un revêtement tel que du TEFLON (nom commercial) déposé sur les surfaces extérieures par projection ou par passage dans un bain.

14. Spéculum monopièce selon la revendication 11 **caractérisé en ce que** la matière plastique est lubrifiée par traitement par plasma froid.

## Patentansprüche

1. Einteiliges, aus Kunststoff geformtes Spekulum mit zwei als Entenschnabel ausgebildeten Spreizarmen (1, 4), welche zu einander schwenkbar sind, wobei jeder der besagten Spreizarme durch ein Greifelement verlängert ist, wobei das Spekulum ferner ein Mittel zur Blockierung der Schwenkung der Spreizarme umfasst, **dadurch gekennzeichnet, dass** die beiden Spreizarme (1, 4) nur durch seitliche Abschnitte (8, 9) verbunden sind, welche sich in der queren Ebene über das hintere Ende der Verbindungsebene (3, 6) der besagten Spreizarme (1, 4) erstrecken und dass einer der Spreizarme (1, 4) mit einem aus einem klappbaren Teil (12) gebildeten Greifelement versehen ist, welches zwischen einer zu der Längsachse des besagten Arms wesentlich senkrechten Formungsstellung und einer Gebrauchsstellung, welche wesentlich in der Längsebene des besagten Arms liegt, beweglich ist.

2. Einteiliges Spekulum nach Anspruch 1, **dadurch gekennzeichnet, dass** der klappbare Teil (12) einen Ausschnitt aufweist, dessen Form das hintere Ende des Spreizarms ergänzt.

3. Einteiliges Spekulum nach Anspruch 1 oder 2, **dadurch gekennzeichnet, dass** der klappbare Teil (12) in der Gebrauchsstellung auf auf der äußeren Fläche des Spreizarms vorgesehenen seitlichen Flügeln abgestützt ist.

4. Einteiliges Spekulum nach einem der vorangehenden Ansprüche, **dadurch gekennzeichnet, dass** einer der Arme (1, 4) durch eine Muffe, welche sich wesentlich senkrecht zu der Achse des Armes erstreckt, verlängert ist.

5. Einteiliges Spekulum nach einem der vorangehenden Ansprüche, **dadurch gekennzeichnet, dass** die Blockierungsmittel aus einer gezahnten Zunge (20, 21) gebildet sind, welche sich von dem Ende einer der Arme in Richtung des Endes des anderen Armes erstreckt, welcher mit einer Stützfläche (22, 23) versehen ist, die geeignet ist, mit der besagten Verzahnung zusammenzuwirken.

6. Einteiliges Spekulum nach Anspruch 5, **dadurch gekennzeichnet, dass** der klappbare Teil (12) eine vorspringende Ecke (24, 25) aufweist, welche geeignet ist, die Blockierungsmittel in Verschlussstellung zu verriegeln, wenn er in Gebrauchsstellung geklappt ist.

7. Einteiliges Spekulum nach Anspruch 6, **dadurch gekennzeichnet, dass** die äußere Fläche der Ecken (24, 25) sich in den Hohlraum (26, 27) einrastet, indem sie in Kontakt mit der Innenfläche des Endes der gezahnten Zungen (20, 21) kommt und dass die Innenfläche (30) der Ecken beim Rückzug auf der Außenfläche (31) des Endes der gezahnten Zunge (20, 21) abgestützt ist, um die Entriegelung zu erleichtern, indem die Stöße bei dem Rückzug des Instruments vermieden werden.

8. Einteiliges Spekulum nach Anspruch 6 oder 7, **dadurch gekennzeichnet, dass** die auf dem klappbaren Teil (12) vorgesehene Ecke (24) eine Rille (48) aufweist, deren Außenkante (47) abgeschrägt ist, um sich auf dem äußeren Rand des Endes (43) der gezahnten Zunge (21) abzustützen und dieses Ende (43) nach Innen hin zu schieben, um die Entriegelung des Armes (1, 4) zu bewirken.

9. Einteiliges Spekulum nach einem der vorangehenden Ansprüche, **dadurch gekennzeichnet, dass** die Spreizarme (1, 4) an dem vorderen Ende eine Ausstülpung (7, 7') aufweisen, welche aus der Innenfläche hervorragt, um ein vollständiges Schließen der besagten Arme zu vermeiden.

10. Einteiliges Spekulum nach einem der vorangehenden Ansprüche, **dadurch gekennzeichnet, dass** die Verbindungsebene (3, 6) der jeweiligen Spreizarme (1, 4) leicht gewölbt ist und eine Verbindungslinie für die Formung aufweist, welche sich im Inneren der Arme (1, 4) befindet.

11. Einteiliges Spekulum nach einem der vorangehenden Ansprüche, **dadurch gekennzeichnet, dass** es aus einem selbstschmierenden Kunststoff besteht.

12. Einteiliges Spekulum nach Anspruch 11, **dadurch gekennzeichnet, dass** der Kunststoff dadurch geschmiert wird, dass in die Masse ein Wirkstoff gemischt wird, welcher zu der Oberfläche des Teils wandert.

13. Einteiliges Spekulum nach Anspruch 11, **dadurch gekennzeichnet, dass** der Kunststoff durch eine Verkleidung wie TEFLON (Handelsname) geschmiert ist, welche durch Spritzen oder dem Durchgang in einem Bad aufgetragen wird.

14. Einteiliges Spekulum nach Anspruch 11, **dadurch gekennzeichnet, dass** der Kunststoff durch Kaltplasmabehandlung geschmiert wird.

## Claims

1. A single-piece speculum moulded in a plastic material, comprising two spacing branches (1, 4) in the form of a duck-bill, which may be pivoted with respect to each other, each of said spacing branches being extended by a prehensile element, the speculum also comprising means for blocking the pivoting of the spacing branches, **characterised in that** the two spacing branches (1, 4) are connected only by two lateral segments (8, 9) extending in the transversal plane passing through the back end of the parting surface (3, 6) of said spacing branches (1, 4), and **in that** one of the spacing branches (1, 4) is fitted with a prehensile element formed by a hingeable part (12) mobile between a moulding position substantially perpendicular to the longitudinal axis of said branch, and a position of use substantially in the longitudinal plane of said branch.

2. The single-piece speculum as claimed in Claim 1, **characterised in that** the hingeable part (12) has a cutout of a complementary shape at the back end of the spacing branch.

3. The single-piece speculum as claimed in Claim 1 or 2, **characterised in that** in the use position the hingeable part (12) is supported on lateral wings provided on the external surface of the spacing branch.

4. The single-piece speculum as claimed in any one of the preceding claims, **characterised in that** one of the branches (1, 4) is extended by a sleeve extending substantially perpendicularly to the axis of the branch.

5. The single-piece speculum as claimed in any one of the preceding claims, **characterised in that** blocking means are formed by a notched tongue (20, 21) extending from the end of one of the branches in the direction of the end of the other branch fitted with a support surface (22, 23) for cooperating with said notchings.

6. The single-piece speculum as claimed in Claim 5, **characterised in that** the hingeable part (12) has a projecting corner (24, 25) for latching the blocking means in a closed position when it is folded down in the use position.

7. The single-piece speculum as claimed in Claim 6, **characterised in that** the external surface of the corners (24, 25) comes to be embedded in the cavity (26, 27), by coming into contact with the inner surface of the end of the notched tongues (20, 21) and **in that**, at the time of withdrawal, the inner surface (30) of the corners (24, 25) comes to rest on the external surface (31) of the end of the notched tongues (20, 21), in order to facilitate unlatching by avoiding jolts when withdrawing the instrument.

8. The single-piece speculum as claimed in Claim 6 or 7, **characterised in that** the corner (24) provided on the hingeable part (12) has a groove (48) whereof the external flank (47) is slanted so as to be able to be supported on the external edge of the end (43) of the notched tongue (21), and to press this end (43) towards the interior so as to cause the branches (1, 4) to be unlatched.

9. The single-piece speculum as claimed in any one of the preceding claims, **characterised in that** the spacing branches (1, 4) have, at their front end, a protuberance (7, 7') which projects relative to the inner surface to avoid complete closing of said branches.

10. The single-piece speculum as claimed in any one of the preceding claims, **characterised in that** the parting surface (3, 6) of the spacing branches respectively (1, 4) is slightly domed and exhibits a moulding joint line located towards the interior of the branches (1, 4).

11. The single-piece speculum as claimed in any one of the preceding claims, **characterised in that** it is constituted by a selflubricated plastic material.

12. The single-piece speculum as claimed in Claim 11, **characterised in that** the plastic material is lubricated by incorporation into the mass an additive migrating towards the surface of the piece.

13. The single-piece speculum as claimed in Claim 11, **characterised in that** the plastic material is lubricated by a coating such as TEFLON (brand name) deposited on the external surfaces by spraying or by passing through a bath.

14. The single-piece speculum as claimed in Claim 11, **characterised in that** the plastic material is lubricated by cold plasma treatment.
